# EUROPEAN PATENT SPECIFICATION

(11) **EP 0 453 242 B1**
(45) Date of publication and mention of the grant of the patent: **21.08.1996**
(21) Application number: 91303387.4
(22) Date of filing: 16.04.1991
(51) Int. Cl.: A61K 48/00, C12N 15/86

(54) **Transfer and expression of gene sequences into central nervous system cells using herpes simplex virus mutants with deletions in genes for viral replication**
Gen-Sequenzentransfer und -exprimierung bei Zellen des zentralen Nervensystems mit mutierenden Herpes-Simplex-Viren, die Deletionen in Viren-Replikationsgenen enthalten
Transfert et expression de séquences géniques dans les cellules du système nerveux central à l'aide de mutants du virus herpès simplex comportant des délétions dans les gènes nécessaires à la réplication virale

(30) Priority: 16.04.1990 US 508731
(43) Date of publication of application: 23.10.1991
(73) Proprietor: THE GENERAL HOSPITAL CORPORATION, Boston, MA 02114 (US)
(72) Inventor: Breakfield,, Xandra O., Newton, Massachusetts 02159 (US); Martuza, Robert L., Lexington, Massachusetts 02173 (US)
(74) Representative: Sheard, Andrew Gregory

(56) References cited:
- WO-A-90/02551
- WO-A-90/09441
- GENE vol. 80, no. 1 Aug. 89, Amsterdam, pages 137-144, Palella et al.
- THE NEW BIOLOGIST. Vol. 2, no. 8, Aug. 90, pages 739-746. Chiocca E. A. et al.

## Description

This invention relates to methods of gene delivery into cells of the central nervous system by introducing and expressing gene sequences using herpes simplex virus 1 (HSV-1) mutants with deletions in gene(s) for viral replication.

The delivery and expression of heterologous or native genes into cells of the nervous system to alter normal cellular biochemical and physiologic processes in a stable and controllable manner is of substantial value in the fields of medical and biological research. This genetic perturbation of the nervous system provides a means for studying the molecular aspects of neuronal function and offering therapeutic approaches to pathologic processes.

Herpes simplex viruses possess several properties that render them attractive candidates as delivery vehicles to bring foreign genes into cells of the peripheral and central nervous systems. (Breakefield, X.O., et al., Mol. Neurobiol. 1:339 (1988); Dobson, A.T., et al., J. Virol. 63:3844-3851 (1989); Ho and Mocarski, Virology 167:279-283 (1988); Ho, D.Y., et al., Proc. Natl. Acad. Sci. USA 86:7596-7600 (1989); Friedmann, T., Science 244:1275-1280; Palella et al., Gene 80:138 (1989).)

These viruses can infect and deliver their DNA into many different cell types, including adult postmitotic neurons; they can enter a state of latency in neurons and possibly other cell types, in which the viral genome exists as an episomal element in the nucleus of the cell; they are able to infect a substantial number of cells in vivo due to their ability to reach high titers in culture and to propagate in the nervous system and the relatively long half life of virus particles; large exogenous DNA sequences can be inserted into their genomes; and they possess a wide host range. (Long-necker, R., et al., In: Viral Vectors, Gluzman, Y., et al. (eds.), CSH Lab, pp. 68-72 (1988); Roizmann, B., et al., In: Virology, Fields, B. N., et al. ( eds. ), Raven Press, New York, pp. 497-526 (1985); Rock, D.L., et al., Nature 302:523-525 ( 1983 ). )

Recently, an amplicon-type plasmid-vector system based on herpes simplex virus 1 (HSV-1) has been used to achieve relatively stable expression of the lacZ gene in cultured neurons. (Spaete, R.R., et al, Proc. Natl. Acad. Sci. USA 82:694 (1985); Geller, A.I., et al, Science 241:1667 (1988); Geller, A.I., et al., Proc. Natl. Acad. Sci. USA 87:1149 (1990).) In this plasmid vector, the lacZ gene was placed under the control of an HSV-1 immediate-early promoter and packaged using a temperature-sensitive HSV-I helper virus tsK. Coen, D.M., et al., Proc. Natl. Acad. Sci. USA 86:4736 (1989); Leib, D.A., et al., J. Virol. 63:759 (1989); Davison, M.J., et al., J. Gen. Virol. 65:859 (1984).)

Several factors appear to limit the potential uses of herpes viruses for gene transfer to cells in culture and in vivo. These include the relatively frequent occurrence of spontaneous revertants of some mutants; possible recombination events between helper virus, viral sequences in plasmids, and latent sequences; deleterious effects on host cell macromolecular synthesis as a result of viral proteins; alterations in the host cell genome; and possible reactivation of preexisting latent viruses. (Davison, M.J., et al., J. Gen. Virol. 65:859 (1984); Fenwick, M., Compr. Virol. 19:359 (1984); Heilbronn, R., et al., J. Virol. 63:3683 (1989); Kwong, A.D., et al., J. Virol. 63:4834 (1989); Johnson, P.A., et al., 14th International Herpes Workshop abst. (1989).)

In the study of gene expression in neuronal cells in the central nervous system, and the identification of therapies for treating neuronal disease of the central nervous system, a need therefore continues to exist for methods of gene delivery with efficient viral vectors capable of mediating gene transfer into such cells without causing harm to the host animal.

According to the present invention, there is provided a herpes simplex virus 1 (HSV-1) vector comprising a mutation in a gene for viral replication and a gene sequence operably linked to a promoter sequence so that the gene sequence can be expressed in a cell; wherein said gene sequence codes for a pharmaceutically active agent or for an agent which modulates neuronal physiology.

Preferred vectors of the present invention are set out in claims 2 to 7.

Vectors of the present invention can be used in a method for introducing and expressing a gene sequence into a central nervous system cell, the method comprising:
introducing into a central nervous system cell a herpes simplex virus 1 (HSV-1) vector comprising a mutation gene for viral replication and a gene sequence operably linked to a promoter sequence so that the gene sequence will be expressed in the cell; and
expressing the gene sequence.

The mutation in the gene for viral replication is preferably in a gene coding for immediate early genes. This may be a gene that encodes infected cell proteins (ICPs) 0, 4, 22, 27 and/or 47.

However, suitably the mutation in the gene for viral replication is in a gene coding for early genes. In such a case, it is preferred that the early genes encodes thymidine kinase or DNA polymerase.

The invention also relates to a pharmaceutical composition comprising the herpes simplex virus 1 mentioned in the above method and a pharmaceutically acceptable carrier.

The invention additionally relates to the use of a herpes simplex virus 1 as described in the above method for use in medicine.

The invention also relates to the use of a herpes simplex virus 1 (HSV-1) vector having a mutation in a gene for viral replication and having a gene sequence operably linked to a promoter sequence, the vector allowing the gene sequence to be expressed in a central nervous system cell so that the expressed gene product complements a neurological deficiency, in the preparation of an agent for treating a neurological deficiency of the central nervous system.

The present invention additionally encompasses the use of a herpes simplex virus 1 (HSV-1) vector with a mutation in a gene for viral replication and having a gene sequence operably linked to a promoter sequence, the vector allowing the expression of the gene sequence in a neuronal cell to modulate neuronal physiology in the preparation of an agent for modulating neuronal physiology in a neuronal cell.

It will be appreciated that the preferred features and characteristics of a method or uses involving the present invention are interchangeable.

The present invention can be used in a method for introducing gene sequences into cells of the central nervous system using HSV-1 mutants as vectors for gene delivery. The HSV-1 virus is mutated so that it has a deletion(s) in a gene(s) for viral replication. The desired gene sequence to be delivered to the cell is inserted into the mutated HSV-1 such that the gene sequence will be expressed in the host cell. Using this method, a gene sequence is expressed in a cell in the central nervous system by introducing the mutated HSV-1 vector with a mutation in a gene for viral replication and with a gene sequence operably linked to a promoter sequence so that the gene sequence will be expressed in the host cell.

The invention can also be used in a method for treating a neurological deficiency of the central nervous system by expressing a gene sequence, which complements the deficiency, in a cell in the central nervous system by introducing a HSV-1 vector with a mutation in a gene for viral replication and with a gene sequence operably linked to a promoter sequence so that the gene sequence will be expressed in the cell and the expressed gene product complements the deficiency.

The invention is further useful in methods for modulating neuronal physiology by delivery of neuropeptide genes, such as those genes that express proteins or polypeptides to block pain; genes that express growth factors; genes that express proteins or polypeptides to promote regeneration or prolong life-span of a cell; genes that express toxic proteins or polypeptides, for example to kill tumor cells. In the methods for modulating neuronal physiology, the method comprises introducing into a cell a HSV-1 vector with a mutation in a gene for viral replication and with a gene sequence coding for the desired protein or polypeptide operably linked to a promoter sequence so that the gene sequence will be expressed in the cell and upon expression will modulate the neuronal physiology.

Using HSV-1 vectors that have mutations in genes necessary for efficient viral replication, the inventors have discovered methods for introducing and expressing a gene sequence in cells of the central nervous system without toxicity to the host animal.

HSV-1 is a double-stranded DNA virus which is replicated and transcribed in the nucleus of the cell. In virions, HSV-1 vectors are composed of head to tail repeats. (Stow, N.D. et al., Eukaryotic Viral Vectors, Y. Gluzman, Ed. (Cold Spring Harbor Laboratory, Cold Spring Harbor, N.Y., 1982), pp. 199-204; Spaete, R.R. et al., Cell 30:285 (1982).) HSV-1 contains approximately 100 genes and is approximately 150 kilobases in size. Five immediate early ("IE") genes encode infected cell proteins (ICPs) 0, 4, 22, 27, and 47, the major regulatory proteins of the virus. These immediate early proteins then act to regulate the expression of viral proteins of the early (E) and late (L) classes. Proteins of the E class are responsible for viral DNA replication. The late ("L") genes are induced after DNA replication and encode the structural components and enzymes required for assembly of virus particles. When the late genes are induced, transcription of the immediate early genes is reduced.

Post-mitotic neurons harbor HSV-1 in the virus' latent state (Stevens, J.G., Curr. Top. Microbiol. Immunol. 70:31-50 (1975).) Once HSV-1 attains latency, it can be retained for the life of the neuron. Latent HSV-1 is capable of expressing genes. Expression of genes encoded by HSV-1 has been detected by in vitro hybridization in latently infected neurons. Furthermore, HSV-1 is transported both anterogradely and retrogradely in neurons, and can be passed transsynaptically. These properties are especially advantageous in that it suggests that HSV-1 vectors will be capable of reaching targets of interest some distance away from the injection site.

Two lines of evidence suggest that HSV-1 can infect most, if not all, kinds of neurons in the central nervous system. First, following inoculation of HSV-1 in the periphery, a burst of virus production ascends the neuraxis, initially in the sensory or motor neurons innervating the site of inoculation, then in the spinal cord, brain stem, cerebellum, and cerebral cortex (Koprowski, H., In: Persistent Viruses (Stevens, F.G., ed.), pp. 691-699, Academic Press, NY (1978).) Second, attempts to mimic HSV-1 latency in tissue culture with different preparations of neurons have required high temperature, DNA synthesis inhibitors, and antisera directed against HSV-1 virions to prevent a lytic infection for spreading to all the neurons (Wigdahl, B., et al., Proc. Natl. Acad. Sci. USA 81:6217-6201 (1984).)

In the method using vectors of the present invention, HSV-1 vectors with mutations in genes involved in viral replication can be used to effect gene delivery to cells in the central nervous system. As used herein, the term HSV-1 vectors, or mutated HSV-1, is meant to include viral HSV-1 in which the viral sequences have been directly modified so that it may be used for a vector for gene delivery. The term "mutations in genes involved in viral replication" is meant to include gene deletions, gene deficiencies, gene activation, and the like. As stated above, genes that are involved in viral replication that may be mutated in this invention include the five immediate-early (IE) viral genes encoding regulatory infected cell proteins (ICPs) 0, 4, 22, 27, and 47. These genes are described in detail in Longnecker, R., *et al.,* In: *Viral Vectors*, Gluzman, Y., *et al.* (eds.), CSH Lab, pp. 68-72 (1988); Roizmann, B., et al., In: Virology, Fields, B.N., et al. (eds.), Raven Press, New York, pp. 497-526 (1985); Rock, D.L., et al., Nature 302:523-525 (1983).

The early genes that are involved in viral DNA replication that may be mutated and used in this invention include thymidine kinase and DNA polymerase. These early temporal class of expressed HSV-1 genes are described in detail in Longnecker, R., et al., In: Viral Vectors, Gluzman, Y., et al. (eds.), CSH Lab, pp. 68-72 (1988); Roizmann, B., et al., In: Virology, Fields, B.N., et al. (eds.), Raven Press, New York, pp. 497-526 (1985); Rock, D.L., et al., Nature 302:523-525 (1983).

The preferred mutated HSV-1 virus vectors are those that enter latency in neurons and do not reactivate or reactivate very inefficiently.

The present invention concerns a means for in vivo introduction of gene sequences into cells of the central nervous system. The cells of the central nervous system include primarily neurons, but also include neuroglia and other cells. These cells are collectively described herein as "neural or neuronal" cells. Neural cells are described, for example, by Barr, M.L., The Human Nervous System An Anatomic Viewpoint, 3rd. Ed., Harper & Row, NY (1979).

The term "gene sequence," as used herein, is intended to refer to a nucleic acid molecule (preferably DNA). Such gene sequences may be derived from a variety of sources including DNA, cDNA, synthetic DNA, RNA, or combinations thereof. Such gene sequences may comprise genomic DNA which may or may not include naturally occurring introns. Moreover, such genomic DNA may be obtained in association with promoter regions or poly A sequences. The gene sequences of the present invention are preferably cDNA. Genomic DNA or cDNA may be obtained in any of several ways. Genomic DNA can be extracted and purified from suitable cells by means well-known in the art. Alternatively, mRNA can be isolated from a cell and used to produce cDNA by reverse transcription, or other means.

As will be appreciated by one of ordinary skill, the nucleotide sequence(s) of the inserted gene sequence or sequences may be of any nucleotide sequence.

The desired gene sequence is operably linked, and under the control of promoter sequence so that the gene sequence will be expressed in the host cell. The promoter sequence is one that is active during viral latency. A viral promoter active during latency can drive stable expression of foreign genes in sensory neurons. (Dobson, A.T., et al., J. Virol. 63:3844-3851 (1989); Ho, D.Y., et al., Proc. Natl. Acad. Sci. USA 86:7596-7600 (1989).) Since herpes virus is known to be maintained in a latent state in the brain, the promoter for the latency-associated transcript (LAT) may be used to mediate stable gene expression. Further, the promoters for the genes for viral replication can be used as promoters to express the gene sequence.

A viral vector, as that term is used herein, is a nucleic acid molecule (preferably of DNA) in which a gene sequence (which is to be transferred) is fused to a subset of viral sequences which are capable of expressing the gene. Also, the viral vector according to this invention will have a mutation in a gene necessary for efficient viral replication. The viral vector should also not have deleterious effects on the host and should infect cells without a helper virus. The viral sequences and the total genome size is selected such that the vector is capable of being encapsulated in a virus particle and thus be capable of binding to, and introducing its gene sequences into a virus-sensitive host cell.

The HSV-1 mutant viral vectors of the present invention can be obtained by transfecting permissive cells with a mixture of plasmid DNA containing HSV-1 gene deletions and infectious and helper HSV-1 DNA. Stow, N.D. et al., Eukaryotic Viral Vectors, Y. Gluzman, Ed. (Cold Spring Harbor Laboratory, Cold Spring Harbor, N.Y., 1982), pp. 199-204; Spaete, R.R. et al., Cell 30:285 (1982).) The obtained mutated HSV-1 viral vectors can be propagated as viruses in appropriate host cells. To grow most mutants, one must transfect a cell with the mutated (and missing) viral gene to replace the deleted viral replication function.

Injection of the vectors to express the desired gene sequence may be done into the brain or into the spinal cord or into nerve endings in the skin, or blood vessels, ventrification surface, etc.

Gene transfer technology has several applications to neuroscience and neurochemistry. The most immediate applications are, perhaps, in elucidating the process of neural peptides and the functional domains of proteins. Cloned cDNA or genomic sequences for neural proteins can be introduced in vivo in order to study cell type-specific differences in processing and cellular fate. By placing the coding sequences under the control of a strong promoter, a substantial amount of the protein can be made, thus avoiding difficulties in characterizing trace amounts. Furthermore, the specific residues involved in protein processing, intracellular sorting, or biological activity can be determined by mutational change in discrete residues of the coding sequence.

Gene transfer technology can also be applied to provide a method to control expression of a protein and to assess its capacity to modulate cellular events in the central nervous system. Certain functions of neural proteins can be studied in vivo, for example, at different times in development or aging in order to monitor changes in receptor density, cell number, fiber growth, electrical activity, and other relevant properties.

Gene transfer provides a means to study the DNA sequences and cellular factors which regulate expression of neural specific genes. 0ne approach to such a study would be to fuse the regulatory elements to be studied to a particular reporter gene and subsequently assaying for the expression of the reporter gene.

The regulation of gene expression in neuronal cells has been found to have a role in maintaining homeostasis and is believed to have a role in mediating information retention in response to external and internal signals (Black, I.B., et al., Science 236:1263-1268 (1987).) During development, coordinate regulation of gene expression serves to produce a differentiated phenotype, e.g., as in catecholamine metabolism and myelin biosynthesis. Regulation depends on many factors including chromatin structure, DNA methylation, and transacting factors, which respond to phosphorylation, hormones, and other signals. It is a complex process that allows sets of genes to be expressed together or differentially and may involve a combinatorial code of regulatory sequences.

Issues of cellular fate and interactions in the central nervous system can also be addressed by gene transfer. For example, genes which encode histological markers can be introduced into embryonic cells to determine lineage relationships during development and to elucidate neuronal pathways and to follow cell maturation and fate. In addition, genes encoding growth factors, oncogenic proteins, toxic peptides, or other physiologically important proteins, can be introduced into specific areas of the brain and spinal cord to study their effects on cell division, survival, and differentiation. For some studies, gene transfer or gene expression must be restricted to specific cells in the nervous system.

Gene transfer also possesses substantial potential use in understanding and providing therapy for disease states. There are a number of inherited neurologic diseases in which defective genes are known and have been cloned. In some cases, the function of these cloned genes is known. In humans, genes for defective proteins have been identified for (1) lysosomal storage diseases such as those involving β-hexosaminidase (Kornerluk, R.G., et al., J. Biol. Chem. 261:8407-8413 (1986); Myerowitz, R., et al., Proc. Natl. Acad. Sci. USA 82:7830-7834 (1985)) and glucocerebrosidase (Sorge et al., ,Proc. Natl. Acad. Sci. USA 82:5442-5445 (1985): Tsuji, S., et al., N. Engl. J. Med. 316:570-575 (1987)), (2) for deficiencies in hypoxanthine phosphoribosyl transferase activity (the "Lesch-Nyhan" syndrome; Stout et al., Met. Enzymol. 151:519-530 (1987)), (3) for amyloid polyneuropathies (prealbumin; Sasaki, H., et al., Biochem. Biophys. Res. Commun. 125:636-642 (1984), (4) for Alzheimer amyloid (Tanzi, R.E., et al., Science 235:880-884 (1987): Goldgaber, D., et al., Science 235:877-880 (1986)); (5) for Duchenne's muscular dystrophy (uncharacterized muscle protein; Monaco, A.P., et al., Nature 323:646-650 (1987)); and (6) for retinoblastoma (uncharacterized protein expressed in the retina and other tissues, Lee, W.-H., et al., Science 235:1394-1399 (1987); Friend, S.H., et al., Nature 323:643-646 (1986).)

Gene transfer techniques can also be used to study the "shiverer" mutation (myelin basic protein, Roach, A., et al., Cell 42:149-155 (1987); Molineaux, S.M., et al., Proc. Natl. Acad. Sci. USA 83:7542-7546 (1986)) and the "jimpy" mutation (proteolipoprotein, Nave, K.-A., et al., Proc. Natl. Acad. Sci. USA 83:9264-9268 (1986); Hudson, L.D., et al., Proc. Natl. Acad. Sci. USA 84:1454-1458 (1987).)

The above diseases fall into two classes: deficiency states, usually of enzymes, which are inherited in a recessive manner; and unbalanced states, at least sometimes involving structural or regulatory proteins, which are inherited in a dominant manner.

For deficiency state diseases, gene transfer could be used to bring a normal gene into affected tissues for replacement therapy, as well as to create animal models for the disease using antisense mutations. For unbalanced state diseases, gene transfer could be used to create the disease state in a model system, which could be used in efforts to counteract the effect of the imbalance. Thus, the methods using vectors of the present invention permit the treatment of neurological diseases. As used herein, a deficiency state disease is "treated" by partially or wholly remedying the deficiency which causes the deficiency or which makes it more severe. As used herein, an unbalanced state disease is "treated" by partially or wholly remedying the imbalance which causes the disease or which makes it more severe.

The method using vectors of this invention may also be used to modulate normal physiologic processes, e.g., delivery of growth factors or other peptides or enzymes to optimize neural regeneration after injury or prolong cell survival in aging or after toxic insults. In addition, it can be used to regulate transmission across certain synapses, for example, to kill neurons in the pathway, or alter neurons within phenotype by up or down regulation of normal neuropeptides or producing neuropeptide analogs as in pain transmission.

In summary, this invention provides a framework for construction and use of replication deficient HSV-1 vectors that can mediate delivery and expression of genes in neurons and other cells in the brain and spinal cord. Virus mutants which cannot replicate in neurons, but can still enter latency, provide the safest vehicle for delivery and appear optimal for targeted delivery to individual neurons either to effect changes in their physiology or to trace their extensions. Mutants which undergo limited replication can be used to alter the physiology of larger groups of neurons and possibly even through to be transmitted across synapses. Such mutants, however, may be somewhat pathogenic, as cells harbouring productive infections may be killed. Herpes vectors thus offer the potential to alter the physiology of single or groups of neurons to study their function and also provide an avenue of exploration for therapeutic interventions aimed at correcting defects and modulating in neuronal functions.

Having now generally described this invention, the same invention will be described in greater detail by reference to the accompanying drawings and Examples which are included herein for purposes of illustration only and are not intended to be limiting of the invention In the drawings:
Figure 1 shows six photomicrographs illustrating β-galactosidase expression in rat brain cells after injection with herpes simplex virus vectors 7134 and RH105 after 1 day, 3 days, and 14 days;
Figure 2 shows a photomicrograph illustrating β-galactosidase expression in rat brain cells after injection with herpes simplex virus vector GAL4;
Figure 3 is a bar chart showing rat brain areas that contain β-galactosidase expressing cells; and
Figures 4a and 4b show 7134-mediated β-galactosidase gene expression in (a) cerebral cortex, and (b) pyramidal neurons in cerebral cortex, respectively.

### EXAMPLE A

Three mutant viruses were studied to explore the potential of these replication deficient mutants as vectors to deliver genes in CNS cells in vivo.

The first vector, 7134, provided by Drs. Priscilla Schaffer and Weizhong Cai (Dana Farber Cancer Center) possesses lacZ substitutions in both copies of HSV-1 ICP0 gene The construction of this vector is described in detail in Cai, W., et al., J. Virol. 63:4579 (1989). Expression of lacZ in this mutant is regulated by the ICP0 immediate-early promoter. Compared to wild-type HSV-1, ICP0 mutants replicate poorly in a variety of cells in culture and in vivo. These mutants establish latency in sensory neurons of trigeminal ganglia as frequently as wild-type HSV-1, when used to inoculate mouse corneas. Once they have established latency, however, they reactivate very inefficiently. (Leib, D.A., et al., J. Virol. 63:759-768 (1989); Sacks, W.R., et al., J. Virol. 61:829-839 (1987).)

The second vector, GAL4, provided by Dr. Neal DeLuca, possesses lacZ substitutions in both copies of the ICP4 gene. This vector is described in detail in Shepard, A.A., et al., J. Virol. 63:3714 (1989), incorporated herein by reference. Expression of lacZ is controlled by the early promoter for the ICP6 gene. ICP4 mutants do not replicate in culture or when inoculated onto mouse corneas. As a consequence, ICP4 mutants are unable to establish latency efficiently, and cannot reactivate from the latency state. (DeLuca, N.A., et al., J. Virol. 62:732-743 (1988); Leib, D.A., et al., J. Virol. 63:759-768 (1989).)

The third vector, RH105, provided by Drs. Edward Mocarski and Dora Ho (Stanford) contains a lacZ substitution in the thymidine kinase gene. This vector is described in detail in Ho, D.Y., et al., Virology 167:279 (1988), incorporated herein by reference. Expression of this gene is regulated by the ICP4 immediate-early promoter. Thymidine kinase (TK) gene is described in palella et al., Gene 80:138 (1989); Coen, D.M., et al., Proc. Natl. Acad. Sci. USA 86:4736 (1989); Ho, D.Y., et al., Virol. 167:279 (1988). TK mutants can replicate in actively growing cells in culture (presumably by utilizing the endogenous cellular thymidine kinase), but replicate poorly following inoculation peripherally or in nervous system cells into rodents. (Leist, T.P., et al., J. Virol. 63:4976 (1989); Coen, D.M., et al., Proc. Natl. Acad. Sci. USA 86:4736 (1989).) These mutants can enter latency in sensory neurons of peripheral ganglia, but do not reactivate from the latent state. (Leist, T.P., et al., J. Virol. 63:4976 (1989); Coen, D.M., et al., Proc. Natl. Acad. Sci. USA 86:4736 (1989).)

Previous animal studies with similar mutant viruses have shown that they are relatively nonpathogenic. (Ho, D.Y., et al., Virology 167:279 (1988); Coen, D.M., et al., Proc. Natl. Acad. Sci. USA 86:4736 (1989); Leib, D.A., et al., J. Virol. 63:759 (1989).) However, these experiments were based on inoculation of epidermal tissues (corneas or footpads) and histological assessment of infections in the sensory ganglia of the peripheral nervous system.

These three vectors, 7134, GAL4, and RH105, were used to express the Escherichia coli lacZ gene in neuronal cells of rat central nervous system. These vectors possessed lacZ deletional substitutions in the genes encoding the immediateearly viral proteins, ICP0 and ICP4, and the early protein, thymidine kinase, and consequently were compromised or defective in their ability to replicate. In all cases lacZ was placed under immediate-early or early viral promoters active in the early phase of infections. Expression of β-galactosidase was observed in cortical neurons following stereotactic inoculation of these mutant viruses into adult rat brains. All mutant vectors exhibited markedly reduced pathogenesis in animals as compared to wild type HSV-1 KOS strain. Different patterns of expression of the β-galactosidase gene were observed with the different vectors used. Injection of the ICP0 mutant in frontal cortex and caudate produced β-galactosidase expression in a substantial number of cells around the inoculation site and at some distance from it for up to 14 days. The ICP0 vector appeared to have been transported retrogradely to the contralateral cingulate cortex. The ICP4 and thymidine kinase mutants showed only a few cells expressing β-galactosidase cells in brain areas immediately adjacent to the injection tract for a few days after injections. These herpes virus-derived vectors provide an opportunity for the in situ delivery and expression of specific genes in neurons in the central nervous system (CNS) with little adverse effect on animals.

### Example 1

In order to evaluate the potential use of vectors 7134, GAL4, and RH105 for direct gene delivery into CNS cells, the potential pathogenic effects on animals following direct intracerebral inoculations was first assessed. Using 200,000 plaque forming units (PFUs) of 7134, GAL4, RH105, or the wild-type KOS strain of HSV-I in 2 µl volumes, these vectors were injected into the right frontal area of adult rat brains. As a control, equal volumes of medium alone were injected. Rat survival and behavior were then assessed daily for two weeks. Table I shows the survival results for these animals. While all animals survived for three days without apparent abnormalities, seven of eight rats injected with KOS and one of seven rats injected with 7134 exhibited profound behavioral abnormalities. The rats injected with KOS showed either lethargy or marked hyperactivity after 3 days, had frequent seizures within 4-5 days, and died by 6 days. The animal that did not survive the 7134 injection was lethargic and showed a marked decrease in feeding and drinking activities starting approximately 8 days after injection and its death occurred rapidly thereafter. One animal that received an intracerebral injection of 7134 became lethargic and perished after 8 days. At autopsy, foci of β-galactosidase positive neurons were found dispersed throughout the cerebral cortex, particularly in the temporal lobes. This pattern appeared similar to the one seen in the rats that had received intracerebral injections of the wild-type HSV-1 KOS. Since this particular rat had been housed mistakenly in a cage that contained rats which had been injected with wild-type virus, one possible explanation for the widespread distribution of lacZ positive cells in this animal is that it also became infected with wild-type HSV-1 KOS, presumably through some of the blood and serious fluid found at the craniotomy site. This might have created a helper effect allowing for the widespread replication of 7134 in this animal.). Control animals and those injected with GAL4 or RH105 viruses showed no behavioral abnormalities or death over a two-week period. These results indicate that these HSV mutant vectors, GAL4, RH105, and 7134, are relatively nonpathogenic and that behavioral abnormalities do not routinely result from their use in rats. A repeated study with 7134 did not show the anomaly previously exhibited.

### Example 2

After inoculations as described in Example 1, rats were sacrificed one, three and fourteen days after virus inoculations in order to assess the extent of β-galactosidase expression. Brains were processed histochemically and assessed for the presence of β-galactosidase activity. In the presence of the substrate X-gal at alkaline pH, this enzyme yields an intense blue stain throughout the cytoplasm of infected cells. (Turner, D.L., et al., Nature 328:131 (1987); Price, J., et al., Proc. Natl. Acad. Sci. USA 84:156 (1987); Sanes, J.R., et al., EMBO J. 5:3133 (1986).)

With the aid of a stereotactic apparatus, approximately 200,000 PFUs of each mutant virus were injected into the right frontal area of rat brains (stereotactic coordinates: AP +2.7/LAT -0.3/Depth -0.4). Two µl volumes were injected with a Hamilton syringe using stereotactic coordinates. Rats were then monitored daily for abnormalities in feeding and drinking behavior and for level of activity. One, three, and fourteen days after viral injections, animals were anesthetized and sacrificed by intracardiac perfusion with 200 ml of 3% paraformaldehyde in phosphate buffered saline. Anesthesia was obtained by intraperitoneal injection of 0.5 ml-1 ml of a solution that contained 16.2% nembutal, 10% ethanol, 39.6% polyethylene glycol, 166 mM magnesium sulfate and 4.25% chloral hydrate. Brains were then processed over the following 3 days by serially placing them for approximately 24 hours in the same solution which contained increasing concentrations of sucrose (15%, 20%, and 30%). After freezing on dry ice and storage at 70°C, brains were sectioned on a cryostat in 40 M sections. After washing with phosphate buffered saline, secretions were reacted overnight in a solution containing 0.1% X-Gal, 2 mM magnesium chloride, 35 mM potassium ferrocyanide, 35 mM potassium ferrocyanide, 0.1% sodium deoxycholate and 0.1% NP4.0. After washing in phosphate buffered saline, sections were mounted on glass slides and examined by light microscopy.

Numerous cells expressing β-galactosidase were observed one, three and 14 days after stereotactic injection of 7134 into the right front cortex. While most cells expressing β-galactosidase were localized within a 175 µm² and around the injection site at day 1, positive cells were found in a 600 µm² area by day 3. By day 14 there was a diminution in the number of cells expressing β-galactosidase with sparse foci located (100 um²) around the needle track.

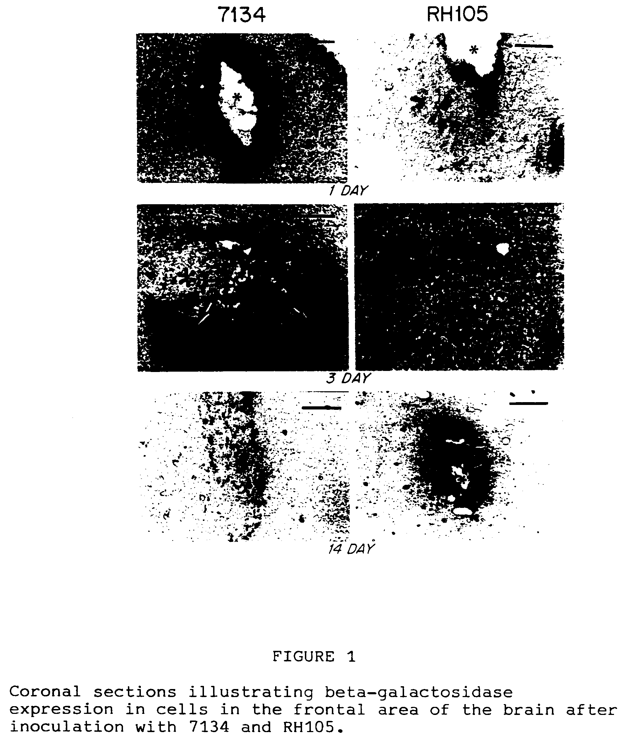

In contrast to the findings for 7134, one and three days after RH105 injection, sparse foci of β-galactosidase expressing cells were present only along the needle track (covering an area of 10 µm²), and by 14 days no positive cells could be detected (Figure 1).

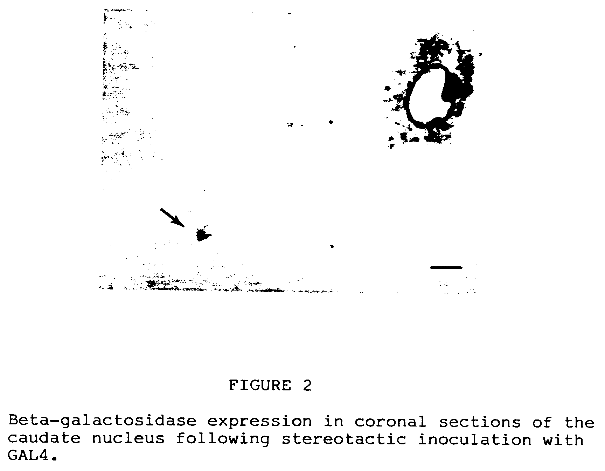

Similarly, after GAL4 injection, only an occasional cell expressing β-galactosidase were observed (Figure 2). Procedures were the same as those given for Figure 1, except that, after the β-galactosidase histochemical reaction, the brain sections were dehydrated in 70% and 80% ethanol and then counterstained with neural red. Sections were then rinsed in 80% ethanol and water before examining them by light microscopy.

These findings indicate that two patterns of gene delivery are characteristic of these vectors. The first, represented by 7134, is that of a replication compromised vector capable of delivering a gene to a relatively large number of cells in the CNS with limited spread away from the site of a delivery. The second, represented by GAL4 and RH105, is that of a replication defective vector that delivers a gene to only a few cells at the injection site without spread to other sites.

The differences in the patterns of gene expression in the CNS among the vectors could also reflect the inactivation of different viral genes, or genetic differences amongst the viral strains used. Vectors 7134 and GAL4 were obtained from an HSV-1 KOS virus strain that had been propagated in one laboratory, whereas RH105 was derived from an HSV-1 KOS strain propagated in a different laboratory. The wild-type HSV-1 KOS strain used as a control in all experiments was also obtained from the same laboratory that vectors 7134 and GAL4 were obtained.

The differences in the patterns of gene expression in the CNS among the vectors may also reflect variations in promoter strength. This latter possibility is not likely, however, since all three promoters, ICP0 (7134), ICP6 (GAL4), and ICP4 (RH105), are expressed with immediate-early kinetics during productive infection.

The pattern of 7134-mediated β-galactosidase expression is best explained by an initial productive infection in numerous cells near the injection site followed by secondary spread of viral particles to adjacent brain areas. Eventual limit to the spread of this virus could be mediated by decreasing yields of virus from infected cells and entry into latency, and/or by immunosuppression. By contrast, the pattern of RH105 or GAL4-mediated β-galactosidase expression indicates that only a small number of cells near the injection site are infected initially, and that no productive infection occurs, although the virus may enter latency.

As controls, brains from rats that had been injected in right frontal areas with the wild-type virus and with medium alone were examined. A monoclonal antibody ID-4 against the major capsid protein of the virus was used to detect infected neurons in rats five days after injection with wild-type HSV-1 KOS. At this time these animals exhibited profound behavioural abnormalities.

Brain sections from HSV-1 KOS injected rats were rinsed in Tris-buffered-saline (TBS) solution (pH 7.4) and incubated for one hour with horse serum (diluted according to Vectastain ABC kit specifications). The sections were then reacted for one hour with medium harvested from the ID-4 monoclonal antibody producing hybridoma line. After thorough rinsing in TBS, the sections were incubated for one hour with biotinylated horse anti-mouse IgG (Vectastain). After additional rinsing, the sections were placed for 30 minutes in Vectastain ABC solution, containing avidin and then in a solution containing 0.01% hydrogen peroxide and 0.05% diaminobenzidine tetrahydrochloride in 0.1 M Tris HCP, pH 7.2 for 2-5 minutes. Sections were then rinsed in TBS and mounted on glass slides.

The immunohistochemical reaction detected numerous areas where viral antigen was present at a distance from the injection site (results not shown). These included bilateral cortical regions in the frontal, cingulate, temporal and parietal lobes. This suggested that the limited number of β-galactosidase expressing cells seen following injection of the mutants is a property of the mutants and is not due to technical artifacts. When medium alone was injected, no blue staining was visible even at higher magnification (results not shown). This control was necessary due to reports that the β-galactosidase histochemical reaction will at times detect endogenous lysosomal β-galactosidase activity. Ho, D.Y., et al., Virology 167:279 (1988).)

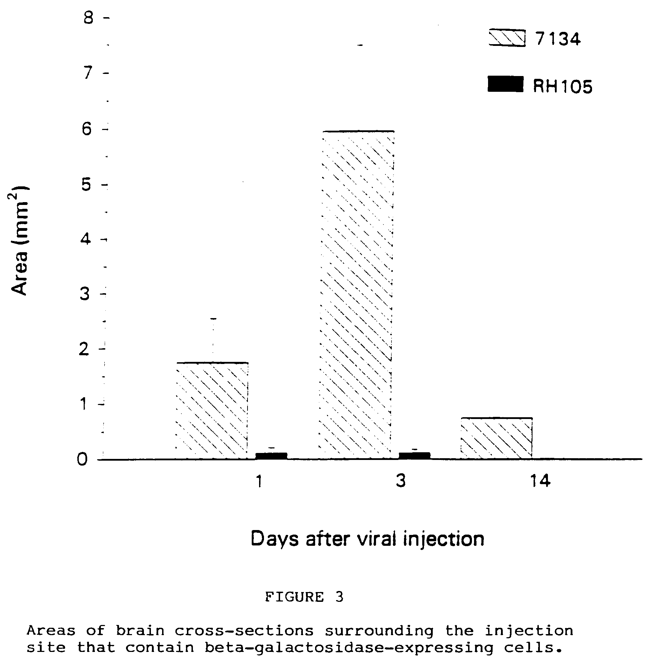

Figure 3 represents rat brain areas that contain β-galactosidase expressing cells. Areas along the needle track were calculated by measuring the furthest linear distance between cells expressing β-galactosidase in two perpendicular directions and then multiplying the two values. Several sections (range: 3-11) were measured for each inoculation from at least two different rats (range: 2-4). Error bars indicate S.D. from mean). Bar graphs represent the averages of these measurements. Measurements were taken with the aid of an ocular micrometer on a light microscope.

To determine which type of cells expressed β-galactosidase, following inoculation of 7134 into the right caudate, sections were then examined for β-galactosidase expression after counterstaining with neutral red. Both small and large types of caudate neurons, as identified by morphology, expressed β-galactosidase three days later.

Stereotactic injections of 7134 were performed in the right caudate nucleus (stereotactic coordinates: AP +1.7; LAT -0.3; Depth -0.4). Animals were sacrificed three days later. Brains were then processed, stained for β-galactosidase activity and counterstained with neutral red as described in the preceding figures.

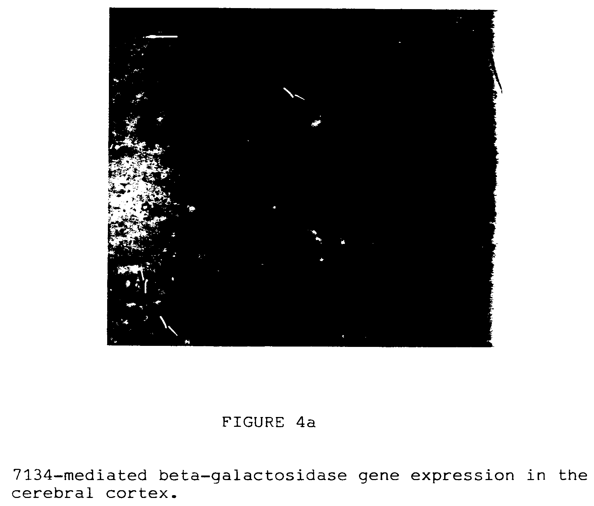

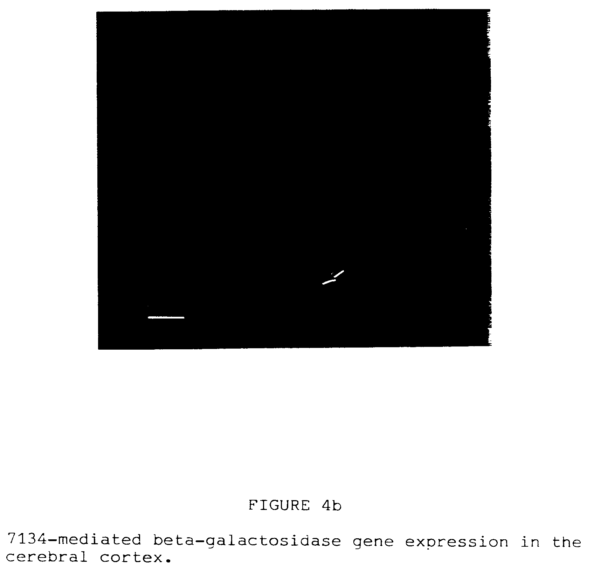

In some rats expression of the lacZ gene was evident in a relatively large area of the cingulate cortex (Figure 4a), probably due to retrograde transport of the virus from the caudate. Examination of the cingulate cortex at higher magnification revealed discrete labeling of pyramidal neurons, identified by characteristic morphology (Figure 4b). Reaction product was apparent throughout dendrites and axons. Most of these neurons did not show evidence of degeneration, such as swelling or beading of processes. It was also evident β-galactosidase gene expression was not as apparent in non-neuronal cell types.

In these studies, lacZ gene transfer and expression into CNS neurons was achieved using replication deficient HSV-1 mutants. The majority of cells expressing lacZ following inoculation of the 7134 replication-compromised mutant possessed the morphological characteristics of neurons. In addition, many of the neurons expressing lacZ, (following caudate inoculation) were found some distance away in the cingulate cortex, raising the possibility that the virus possesses a particular trophism for this cortical region. This finding also supports the occurrence of retrograde transport of the mutant virus following from nerve endings in the caudate to cell bodies in the cortex. Transneuronal transport can deliver genes to specific neurons via inoculation into areas containing their axonal or dendritic projections. Recent findings using wild type HSV-1 indicate that transneuronal and transsynaptic transfer can be followed using ³H-thymidine labeling and immunocytochemical staining of viral antigens. (Ugolini, G., et al., Science 243:89 (1989); Norgren, Jr., R.B., et al., Brain Res. 479:374-378 (1989); Margolis, T.P., et al., J. Virol. 63:4756-4761 (1989); Kuypers, G.J.M., et al., TINS 13:71-75 (1990).) The viral vectors with the lacZ substitutions thus should be used for transsynaptic transport. This provides a useful tool to neuroanatomically trace specific neuronal projections and synaptic pathways with less toxicity to animals.

**TABLE 1**

| SURVIVAL OF RATS AFTER INTERCEREBRAL INJECTION OF VIRUS | | | |
|---|---|---|---|
| | Days After Injection | | |
| Mutant | 1 | 3 | 14 |
| Control^{a} | 12/12^{b} | 8/8 | 4/4 |
| 7134 | 26/26 | 8/8 | 6/7^{c} |
| 7134 | 12/12 | 12/12 | 12/12 |
| GAL4 | 15/15 | 11/11 | 3/3 |
| RH105 | 12/12 | 8/8 | 4/4 |
| KOS | 19/19 | 15/15 | 1/8^{d} |

| | | | |
|---|---|---|---|
| ^{a}Equal volume of medium was injected. | | | |
| ^{b}Expressed as animals surviving/animals inoculated on day 0. | | | |
| ^{c}Death of one animal occurred 8 days after injection. This animal exhibited lethargy and inactivity prior to death. | | | |
| ^{d}Animal deaths occurred 6-7 days after inoculation and were preceded by frequent seizures 5 days following inoculation. | | | |

## Claims (Claims for the following Contracting State(s): AT, BE, CH, DE, DK, GB, FR, IT, LI, LU, NL, SE)

1. A herpes simplex virus 1 (HSV-1) vector comprising a mutation in a gene for viral replication and a gene sequence operably linked to a promoter sequence so that the gene sequence can be expressed in a cell; wherein said gene sequence codes for a pharmaceutically active agent or for an agent which modulates neuronal physiology.

2. A vector according to claim 1 wherein the mutation in the gene for viral replication is in a gene coding for immediate early genes.

3. A vector according to claim 2 wherein the gene coding for immediate early genes is a gene that encodes infected cell proteins (ICPs) 0, 4, 22, 27 and/or 47.

4. A vector according to claim 1 wherein the mutation in the gene for viral replication is in a gene coding for early genes.

5. A vector according to claim 4 wherein the gene coding for early genes encodes thymidine kinase or DNA polymerase.

6. A vector according to any preceding claim wherein said gene sequence codes for: a neuropeptide, a growth factor, a protein or polypeptide which promotes regeneration or prolongs the life-span of a cell, a toxic protein or polypeptide, a gene product which complements a neurological deficiency of the central nervous system, a histological marker, or an oncogenic protein.

7. A vector according to any preceding claim for use in medicine.

8. The use of a herpes simplex virus 1 (HSV-1) vector according to any preceding claim in the preparation of an agent for treating a neurological deficiency of the central nervous system.

9. The use of a herpes simplex virus (HSV-1) vector according to any of claims 1 to 7 in the preparation of an agent for modulating neuronal physiology in a neuronal cell.

10. A pharmaceutical composition comprising a vector according to any of claims 1 to 7 and a pharmaceutically acceptable carrier.

## Claims (Claims for the following Contracting State(s): ES, GR)

1. A method for providing a herpes simplex virus 1 (HSV-1) vector in which there is provided a mutation in a gene for viral replication and a gene sequence operably linked to a promoter sequence so that the gene sequence can be expressed in a cell; wherein said gene sequence codes for a pharmaceutically active agent or for an agent which modulates neuronal physiology.

2. A method according to claim 1 wherein the mutation in the gene for viral replication is in a gene coding for immediate early genes.

3. A method according to claim 2 wherein the vector comprises a gene coding for immediate early genes is a gene that encodes infected cell proteins (ICPs) 0, 4, 22, 27 and/or 47.

4. A method according to claim 1 wherein the mutation in the gene for viral replication is in a gene coding for early genes.

5. A method according to claim 4 wherein the gene coding for early genes encodes thymidine kinase or DNA polymerase.

6. A method according to any preceding claim wherein said gene sequence codes for: a neuropeptide, a growth factor, a protein or polypeptide which promotes regeneration or prolongs the life-span of a cell, a toxic protein or polypeptide, a gene product which complements a neurological deficiency of the central nervous system, a histological marker, or an oncogenic protein.

7. A method according to any preceding claim for use in medicine.

8. The use of a herpes simplex virus 1 (HSV-1) vector as described in any preceding claim in the preparation of an agent for treating a neurological deficiency of the central nervous system.

9. The use of a herpes simplex virus (HSV-1) vector as described in any of claims 1 to 7 in the preparation of an agent for modulating neuronal physiology in a neuronal cell.

10. A method of producing a pharmaceutical composition comprising combining a vector as described in any of claims 1 to 7 and a pharmaceutically acceptable carrier.

## Patentansprüche (Patentansprüche für folgende(n) Vertragsstaat(en): AT, BE, CH, DE, DK, GB, FR, IT, LI, LU, NL, SE)

1. Herpes-simplex-Virus-1 (HSV-1)-Vektor, welcher eine Mutation in einem Gen für virale Replikation und eine Gensequenz umfaßt, die funktionell mit einer Promotor-Sequenz verbunden ist, so daß die Gensequenz in einer Zelle exprimiert werden kann, wobei die Gensequenz für einen pharmazeutisch aktiven Wirkstoff oder für einen Wirkstoff codiert, der die neuronale Physiologie moduliert.

2. Vektor nach Anspruch 1, wobei sich die Mutation in dem Gen für virale Replikation in einem Gen befindet, das für unmittelbare frühe Gene codiert.

3. Vektor nach Anspruch 2, wobei das für unmittelbare frühe Gene codierende Gen ein Gen ist, das für die infizierten Zellproteine (ICPs) 0, 4, 22, 27 und/oder 47 codiert.

4. Vektor nach Anspruch 1, wobei sich die Mutation in dem Gen für virale Replikation in einem Gen befindet, das für frühe Gene codiert.

5. Vektor nach Anspruch 4, wobei das für frühe Gene codierende Gen Thymidinkinase oder DNA-Polymerase codiert.

6. Vektor nach irgendeinem der vorhergehenden Ansprüche, wobei die Gensequenz für ein Neuropeptid, einen Wachstumsfaktor, ein Protein oder Polypeptid, das die Regeneration einer Zelle fördert oder deren Lebenszeit verlängert, ein toxisches Protein oder Polypeptid, ein Genprodukt, das einen neurologischen Mangel des Zentralnervensystems ausgleicht, einen histologischen Marker oder ein onkogenes Protein codiert.

7. Vektor nach irgendeinem der vorhergehenden Ansprüche zur Verwendung in der Medizin.

8. Verwendung eines Herpes-simplex-Virus-1 (HSV-1)-Vektors nach irgendeinem der vorhergehenden Ansprüche bei der Herstellung eines Mittels zur Behandlung eines neurologischen Mangels des Zentralnervensystems.

9. Verwendung eines Herpes-simplex-Virus-1 (HSV-1)-Vektors nach einem der Ansprüche 1 bis 7 bei der Herstellung eines Mittels zur Modulation der neuronalen Physiologie in einer Nervenzelle.

10. Pharmazeutische Zusammensetzung, die einen Vektor nach einem der Ansprüche 1 bis 7 und einen pharmazeutisch annehmbaren Träger enthält.

## Patentansprüche (Patentansprüche für folgende(n) Vertragsstaat(en): ES, GR)

1. Verfahren zur Bereitstellung eines Herpes-simplex-Virus-1 (HSV-1)-Vektors, bei dem eine Mutation in einem Gen für virale Replikation vorgesehen ist, sowie einer Gensequenz, die funktionell mit einer Promotor-Sequenz verbunden ist, so daß die Gensequenz in einer Zelle exprimiert werden kann, wobei die Gensequenz für einen pharmazeutisch aktiven Wirkstoff oder für einen Wirkstoff codiert, der die neuronale Physiologie moduliert.

2. Verfahren nach Anspruch 1, wobei sich die Mutation in dem Gen für virale Replikation in einem Gen befindet, das für unmittelbare frühe Gene codiert.

3. Verfahren nach Anspruch 2, wobei der Vektor ein Gen umfaßt, das für unmittelbare frühe Gene codiert und ein Gen ist, das die infizierten Zellproteine (ICPs) 0, 4, 22, 27 und/oder 47 codiert.

4. Verfahren nach Anspruch 1, wobei sich die Mutation in dem Gen für virale Replikation in einem Gen befindet, das für frühe Gene codiert.

5. Verfahren nach Anspruch 4, wobei das für frühe Gene codierende Gen Thymidinkinase oder DNA-Polymerase codiert.

6. Verfahren nach irgendeinem der vorhergehenden Ansprüche, wobei die Gensequenz für ein Neuropeptid, einen Wachstumsfaktor, ein Protein oder Polypeptid, das die Regeneration einer Zelle fördert oder deren Lebenszeit verlängert, ein toxisches Protein oder Polypeptid, ein Genprodukt, das einen neurologischen Mangel des Zentralnervensystems ausgleicht, einen histologischen Marker oder ein onkogenes Protein codiert.

7. Verfahren nach irgendeinem der vorhergehenden Ansprüche zur Verwendung in der Medizin.

8. Verwendung eines Herpes-simplex-Virus-1 (HSV-1)-Vektors nach irgendeinem der vorhergehenden Ansprüche bei der Herstellung eines Mittels zur Behandlung eines neurologischen Mangels des Zentralnervensystems.

9. Verwendung eines Herpes-simplex-Virus-1 (HSV-1)-Vektors nach einem der Ansprüche 1 bis 7 bei der Herstellung eines Mittels zur Modulation der neuronalen Physiologie in einer Nervenzelle.

10. Verfahren zur Herstellung einer pharmazeutischen Zusammensetzung, das die Kombination eines Vektors nach einem der Ansprüche 1 bis 7 mit einem pharmazeutisch annehmbaren Träger umfaßt.

## Revendications (Revendications pour l'(les) Etat(s) contractant(s) suivant(s): AT, BE, CH, DE, DK, GB, FR, IT, LU, NL, SE)

1. Vecteur du virus Herpes simplex 1 (HSV-1) comprenant une mutation dans un gène nécessaire à la réplication virale et une séquence génique liée de manière opérable à une séquence promoteur, de sorte que la séquence génique puisse être exprimée dans une cellule; où la séquence génique code pour un agent pharmaceutiquement actif ou pour un agent qui module la physiologie neuronale.

2. Vecteur suivant la revendication 1, dans lequel la mutation dans le gène nécessaire à la réplication virale se situe dans un gène codant pour des gènes précoces immédiats.

3. Vecteur suivant la revendication 2, dans lequel le gène codant pour les gènes précoces immédiats est un gène qui code les protéines de cellule infectée (PCI) 0, 4, 22, 27 et/ou 47.

4. Vecteur suivant la revendication 1, dans lequel la mutation dans le gène nécessaire à la réplication virale se situe dans un gène codant pour des gènes précoces.

5. Vecteur suivant la revendication 4, dans lequel le gène codant pour les gènes précoces code la thymidine kinase ou l'ADN polymérase.

6. Vecteur suivant l'une quelconque des revendications précédentes, dans lequel la séquence génique code pour : un neuropeptide, un facteur de croissance, une protéine ou un polypeptide qui favorise la régénération ou prolonge la durée de vie d'une cellule, une protéine ou un polypeptide toxique, un produit génique qui complémente une déficience neurologique du système nerveux central, un marqueur histologique ou une protéine oncogène.

7. Vecteur suivant l'une quelconque des revendications précédentes, à utiliser en médecine.

8. Utilisation d'un vecteur du virus Herpes simplex 1 (HSV-1) suivant l'une quelconque des revendications précédentes, dans la préparation d'un agent pour le traitement d'une déficience neurologique du système nerveux central.

9. Utilisation d'un vecteur du virus Herpes simplex (HSV-1) suivant l'une quelconque des revendications 1 à 7, dans la préparation d'un agent pour moduler la physiologie neuronale d'une cellule neuronale.

10. Composition pharmaceutique comprenant un vecteur suivant l'une quelconque des revendications 1 à 7 et un excipient pharmaceutiquement acceptable.

## Revendications (Revendications pour l'(les) Etat(s) contractant(s) suivant(s): ES, GR)

1. Procédé fournissant un vecteur du virus Herpes simplex 1 (HSV-1), dans lequel on introduit une mutation dans un gène nécessaire à la réplication virale et une séquence génique liée de manière opérable à une séquence promoteur, de sorte que la séquence génique puisse être exprimée dans une cellule; où la séquence génique code pour un agent pharmaceutiquement actif ou pour un agent qui module la physiologie neuronale.

2. Procédé suivant la revendication 1, dans lequel la mutation dans le gène nécessaire à la réplication virale se situe dans un gène codant pour des gènes précoces immédiats.

3. Procédé suivant la revendication 2, dans lequel le vecteur comprend un gêne codant pour les gènes précoces immédiats, qui est un gène qui code les protéines de cellule infectée (PCI) 0, 4, 22, 27 et/ou 47.

4. Procédé suivant la revendication 1, dans lequel la mutation dans le gène nécessaire à la réplication virale se situe dans un gène codant pour des gènes précoces.

5. Procédé suivant la revendication 4, dans lequel le gène codant pour les gènes précoces code la thymidine kinase ou l'ADN polymérase.

6. Procédé suivant l'une quelconque des revendications précédentes, dans lequel la séquence génique code pour : un neuropeptide, un facteur de croissance, une protéine ou un polypeptide qui favorise la régénération ou prolonge la durée de vie d'une cellule, une protéine ou un polypeptide toxique, un produit génique qui complémente une déficience neurologique du système nerveux central, un marqueur histologique ou une protéine oncogène.

7. Procédé suivant l'une quelconque des revendications précédentes, à utiliser en médecine.

8. Utilisation d'un vecteur du virus Herpes simplex 1 (HSV-1) comme décrit dans l'une quelconque des revendications précédentes, dans la préparation d'un agent pour le traitement d'une déficience neurologique du système nerveux central.

9. Utilisation d'un vecteur du virus Herpes simplex (HSV-1) comme décrit dans l'une quelconque des revendications 1 à 7, dans la préparation d'un agent pour moduler la physiologie neuronale d'une cellule neuronale.

10. Procédé de préparation d'une composition pharmaceutique comprenant la combinaison d'un vecteur comme décrit dans l'une quelconque des revendications 1 à 7 et d'un excipient pharmaceutiquement acceptable.
